# EUROPEAN PATENT SPECIFICATION

(11) **EP 1 562 646 B1**
(45) Date of publication and mention of the grant of the patent: **05.04.2006**
(21) Application number: 03780649.4
(22) Date of filing: 24.11.2003
(51) Int. Cl.: A61L 9/16, A61L 9/22, B01D 53/32, F24F 3/16

(54) **METHOD AND DEVICE FOR THE SANITIZATION OF AIR**
METHODE UND VORRICHTUNG FÜR DIE REINIGUNG VON LUFT
PROCEDE ET DISPOSITIF DE PURIFICATION DE L'AIR

(30) Priority: 22.11.2002 IT VI20020255
(43) Date of publication of application: 17.08.2005
(73) Proprietor: Mertens, Daniel, 36051 Creazzo (IT); Amendola, Giuseppe, 31044 Montebelluna (IT); Pertegato, Giancarlo, 36100 Vicenza (IT)
(72) Inventor: Mertens, Daniel, 36051 Creazzo (IT); Amendola, Giuseppe, 31044 Montebelluna (IT); Pertegato, Giancarlo, 36100 Vicenza (IT)
(74) Representative: Gallo, Luca
(86) International application number: PCT/IT2003/000770
(87) International publication number: WO 2004/047877

(56) References cited:
- WO-A-02/17978
- DE-A- 3 634 538
- US-A- 4 244 712
- US-A- 5 656 063

## Description

The present invention relates to a method and a device for the environmental and localized sanitization of air and similar gaseous mixtures and of all the surfaces and substances in contact therewith, applicable during the production, processing, packaging and conservation of products under controlled contamination and aseptic conditions, as well as in all those situations where environmental contamination control is required.

In the text of the present description and in the following claims, "air" is understood as referring to the gaseous mixture of nitrogen and oxygen with small quantities of other gases, which form the atmosphere of the earth as well as all the similar gaseous mixtures which may also contain solid and/or liquid elements in suspended particle form.

It is known that the contamination of products or environments with a high level of purity, associated with the food, pharmaceutical, herbal, sanitary, cosmetics, electronics and industrial sectors, occurs as a result of the action of microbes and extremely fine particles of dust which are present in the air, on the persons who may handle said products and on the surfaces with which they come into contact, and also as a result of the residual bacterial content which may be present in the products themselves or also owing to mixed contamination. The latter case is understood as referring to the situation where products with a typical bacterial content are mistakenly processed without isolating systems in the vicinity of other products which have a different typical bacterial content or where different processing steps are mistakenly performed under mixed conditions. In said cases, contaminating factors which are not harmful for certain products and processes may be transferred to other products and production lines where they may instead produce chemical and physical or organoleptic alterations or adversely affect the healthy character or purity of the product. This concept is understood as being applicable also to the case of chemical contamination due to particles suspended in the air or in air-like medium in which the processing operations are performed.

It is also known that, in order to increase the safety of the products for human or animal use or consumption, prolong their shelf life or eliminate possible sources of pollution for products which require a high degree of purity, it is necessary to improve as far as possible environmental control methods in all their aspects.

A first method currently used to sanitize the air in controlled contamination environments is the use of mechanical filtration systems with which absolute degrees of filtration of the air may be obtained. The drawbacks associated with this type of sanitization system are numerous. Firstly these systems form a zone harbouring and promoting the growth of microbes and liable to release high levels of contamination during replacement of the filters, with consequent prolonged contamination of the environments to be protected. Since the replacement operations must be performed regularly in view of the gradual blockage of the mechanical filters, the contamination processes occur repeatedly during the course of normal maintenance of the filters. Moreover, this system does not allow continuous decontamination of the filtration devices and the treated environments and requires large amounts of power in order to overcome the resistance created by the filter structure to the passage of air. Finally the concentration of microbes which occurs on the surfaces before the filters causes the passage of toxic substances due to metabolism of the microbes or decomposition thereof; these substances (endotoxins) are inevitably spread downstream of the filter if the latter is not replaced in the due manner and time. To conclude, this system is a passive system which is unable to reduce or eliminate the microbial contamination accumulated, does not use methods which act directly on the harmful microorganisms conveyed in the air and does not act directly on the conditions of the environments into which the filtered air is emitted. In an attempt to overcome the problems of contamination of the apparatus, washable apparatus for treatment and conditioning of the air in processing and packaging environments have been introduced. The drawback of washing is that it does not solve definitively the problem of contamination. The periodic washing of these apparatus using detergents and disinfectants has provided partial and temporary results since the reoccurrence of contamination and the diffusion of the microorganisms during the processing and operating cycle is not prevented. These apparatus are therefore still classifiable as "passive" in terms of their action on microbiological flora. Sanitization by means of washing, which must be regular and very thorough in order the avoid the accumulation of a microbial content, results in production downtime and requires a specific cleaning and maintenance cycle.

Other solutions proposed for decontaminating air are based from an operational point of view on the use of ozone or ultraviolet rays or on the use of the positive and/or negative ions of the air. The sanitization methods in this case are of the active and continuous type: the microbes are not merely confined and retained by means of a mechanical system, but the termination of the vital functions of the microorganisms is caused in a continuous manner wherever the sanitization process is able to act. Decontamination incorporated within the air treatment cycle thus becomes an "active" method. These methods have the effect of reducing the action of the microbial content not only in the treated air but also in the environments where this treated air is confined. Although achieving good results for the different varieties of microorganisms, the drawbacks of the "active" sanitization methods which may be encountered hitherto on the market are notable and limit the use thereof.

The drawbacks of ozone as a germicidal agent have been known for some time. The use of this gas poses serious problems associated with its toxicity and the reactivity of the molecule which causes superficial oxidation phenomena, discolouring, corrosion, irritation, etc. It is also known that, in order to be effective for the purposes of air decontamination, it is required to reach ozone concentrations which exceed the TLV (Threshold Limit Value) adopted by the ACGIH (American Conference of Governmental Industrial Hygienists) and subsequently by the AIDII (Italian Association of Industrial Hygienists), which fixes the concentration in working environments at a maximum of 0.1. ppm based on an 8-hour working day and 40-hour week. Ozone therefore cannot be used effectively as a germicidal agent in environments and premises where the presence of working personnel is envisaged. In other environments, where operators are not envisaged, the use of ozone in higher concentrations causes the deterioration of various substances, in particular synthetic materials.

The second active method for sanitizing air, which is based on the use of ultraviolet rays (UV), has a major mutagenic effect on the microorganisms, but in this case there are drawbacks which make the method unsuitable for treatment on an industrial level. The effect of ultraviolet rays is evident only on microorganisms which are directly struck by the radiation and for a sufficiently long period of time. It is therefore impossible to use this method to sanitize environments or devices where the rays are unable to reach all the spaces which may be contaminated. It is equally impossible to sanitize effectively the air with ultraviolet rays when recirculation thereof is required in order to ensure a certain number of hourly change-overs in the environments and premises, as occurs in most air-conditioning and treatment plants. The contact times in this case are too short. In addition to these limitations, another drawback consists in the fact that the UV lamps over time lose their power and require frequent checks which thus render the system unsuitable for installation in closed spaced such as the conveying ducts or pipes of air-conditioning plants. Finally, persons may suffer from conjunctivitis and erythema if exposed directly to UV rays.

The third active method mentioned for sanitizing air, namely the use of air ionization for sanitization purposes, is a method which is widely used for the construction of decontamination systems in the civil sector and in the home environment and is based on the oxidizing properties of the air ions. In 2001 patent of industrial invention No. 01307086 was granted in Italy, said patent relating to a "Method and device for the localized sanitization of air and similar gaseous mixtures" based on the germicidal properties of a gaseous mixture of negative air ions, emitted into the air to be sanitized. Subsequently European patent application No. 00830334.9 was filed, said application having an object similar to that of patent No. 01307086, but associating with ionization of the air, electrostatic filtration thereof for the purposes of purification and sanitization, both of a general (environmental) and localized nature. The principles used, i.e. electrostatic filtration and air ionization, rigorously avoid the production of other gases, such as ozone, which is explicitly and specifically excluded in both methods described and in the claims of both inventions. The combination of electrostatic filtration and the anti-microbic effect of the gaseous mixture of the negative air ions without the production of ozone result in the purification of air with results which are not entirely satisfactory. The two principles are considered separately and a particular functional synergy between them is not envisaged. The invention according to patent of industrial invention No. 01307086 and that of European patent application No. 00830334.9 have a series of limitations which the method and device illustrated below intend to overcome.

Electrostatic filtration is unable to achieve, using economically competitive devices, a filtration efficiency which is greater than the level H12 according to the standard EN 1822, necessary for environments with a controlled contamination category less than or equal to 100,000 particles per cubic foot, with particles having dimensions less than or equal to 0.5 µm.

As regards air ionization, experimental research carried out by the Applicant has shown that the sanitization systems which work exclusively with the air have only slight bactericidal effects on certain microorganisms and bacterially static effects on other families of germs and their spores. The efficiency in reduction of the microbial content due to the positive and/or negative air ions moreover diminishes considerably when the relative humidity of the air reaches values which exceed 90% or when it reaches levels which are often necessary in some processing operations, such as those of numerous food products.

In connection with these analyses, it is also necessary to take into account the scientific research conducted in Japan by Yasuhiro Tanimura, Junji Hirotsuji and Takao Yamayoshi and published in 1999 in the "JOURNAL OF ANTIBACTERIAL AND ANTIFUNGAL AGENTS", an authoritative scientific journal of this country. The researchers have in fact shown how a gaseous mixture of positive ions and/or negative air ions and low concentration ozone have lethal effects on *Escherichia coli* and *Staphylococcus aureus*, which are substantially greater than those which can be obtained with the separate use of the individual components of the gaseous mixture. Similarly, further experiments conducted by the Applicant by way of verification and amplification of the research carried out in Japan, on two types of fungal spores: *Aspergillus niger* and *Mucor sporigenum* (also called *Mucor javanicus*) have confirmed the results obtained on different types of bacteria in Japan, demonstrating percentage reductions with the use of a gaseous mixture of positive and/or negative air ions and low concentration ozone markedly greater than those obtained with the single and separate use of the negative air ions and ozone, even in notably higher concentrations.

This research has clearly demonstrated that the mixing together of negative air ions and low concentration ozone has a synergic effect, increasing and expanding the anti-microbic power of the individual components, which is practically doubled compared to the result which can be obtained with the single and separate use thereof, with the same concentrations and under the same temperature and humidity conditions. The mixture of negative air ions and low concentration ozone has an anti-microbic effect such as to ensure a major reduction in the bacterial and fungal content both directly in the air (environmental application) and on all the surfaces which come into contact with said mixture (localized application), including the abovementioned products (associated with the food, pharmaceutical, herbal, sanitary, cosmetics, electronics and industrial sectors). In addition to its notable anti-microbic properties, the main advantage of this research is that of achieving this result by using a mixture which does not negatively affect the health of the operators or damage the materials with which it comes into contact, in view of the low concentration of ozone involved and the non-toxicity of the air ions for human health.

It is also known from patent US 4,244,712 an air cleaning system comprising a portable housing with a blower means to draw air through the housing, which incorporates an electrostatic air cleaner, a charcoal filter, an ozone gas generator and a negative ion generator to provide treated air having ozone and negative ions and wherein the electrostatic cleaner and charcoal filter physically and chemically remove impurities.

The main object of the present invention is that of overcoming the drawbacks and limitations of the existing systems by providing a method and a device for the environmental and localized sanitization of air, able to reduce or eliminate much more significantly than in the past the microbic content contained therein and purify it from the point of view of its particle content within a confined environment.

Within the context of this specific task, one object of the invention is to use in a synergically integrated manner known sanitization methods so as to reduce the microbic content in a low-cost and effective manner.

A further object of the invention is that of providing a method and a device which allow the prevention and reduction of air-transported particle and microbiological pollution. Said method and device involve, on the one hand, the exercising of a germicidal action for environmentally sanitizing and purifying all the air introduced into a confined environment and, on the other hand, using the method in a localized manner, as a solution for providing controlled contamination or stable aseptic conditions on surfaces or in restricted spaces.

In addition to sanitization obtained in the air, another object of the invention is that of maintaining and improving the hygienic conditions of all the surfaces with which the treated air comes into contact.

Another object of the invention is that of providing a method and a device which act directly on the surface of the products which are perishable and/or subject to contamination, reducing their microbic content so as to prolong their shelf life as well as maintain their healthy character and organoleptic properties, in the case of food products, also when combining the method with known methods.

Another object of the invention is that of providing a method and device for sanitizing foodstuffs which may be implemented in synergy with other methods, processes and devices already known for the treatment, conservation and/or maturation of certain food products. In this context, the method and device of the invention may be combined with processes such as those listed hereinbelow by way of a non-limiting example, namely processes for refrigeration, curing, desiccation, dehydration, drying, dehumidification, aeration, curing, emulsification, peeling and stripping, in order to optimize the efficiency from the microbiological and organoleptic point of view using these already known methods.

Another object of the invention is that of providing a sanitization method and device which allow a drastic reduction in the operating costs per cubic metre of air to be treated for the purposes of sanitization.

Last but not least, an object of the invention is to provide a method and device which allow a substantial reduction in the cost of maintenance of air treatment and conveying plants, limiting the cleaning operations and eliminating periodic washing, with implementation of a sanitizing system which is continuously active during the normal treatment cycle.

The task indicated, together with these and other objects which will be clarified more fully below, are achieved by the method for the environmental and localized sanitization of air, described hereinbelow and illustrated solely by way of a non-limiting example in the descriptions and in the accompanying illustrative plates in which:
- Figure 1 shows a schematic view of an example of a device for environmental sanitization according to the invention;
- Figure 2 shows a schematic side view of an example of a device for localized sanitization according to the invention, for curing warehouses;
   - Figure 2a shows a first detailed view of an air ionization apparatus, said element forming part of the device shown in Figure 2;
   - Figure 2b shows a plan view of the device shown in Figure 2;
   - Figure 2c shows a side view of a portion of the device according to Figure 2;
- Figure 3 shows a schematic view of an example of a device for localized sanitization according to the invention, inside a transfer tunnel.

The present invention relates to a method for the environmental or localized sanitization of air.

The method is intended for the sanitization, or reduction of the microbiological or particle contamination of a confined environment by means of the removal of the said microbiological or particle content from an air flow which affects the said confined environment.

The latter may be formed by an air conveying plant, by its ducts or by environments which are suitable for receiving persons (working premises, residential premises or the like) or products (warehouses) and are for example connected to said conveying plant, or may consist more specifically in apparatus or machinery for the treatment of products, in particular perishable products. In accordance with this latter intended use, this method may be envisaged for the sanitization of confined environments consisting of cooling rooms, booths, sterile rooms, storage tanks, silos, vats or the like.

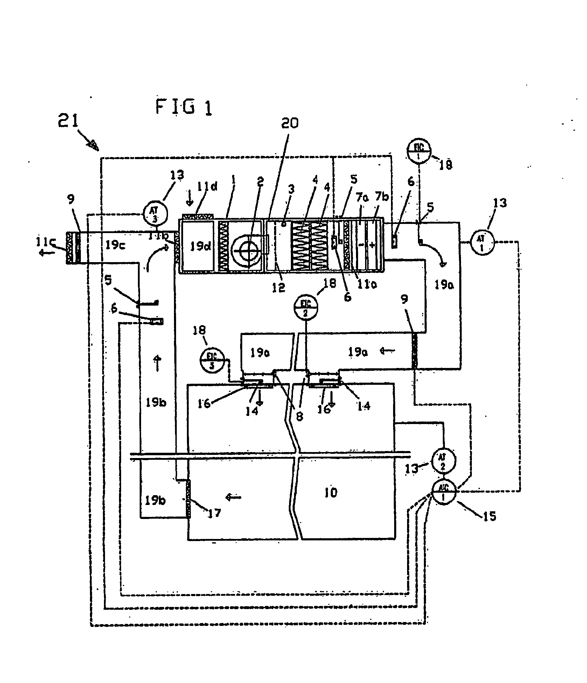

In any case the method will be advantageously associated with an air treatment unit denoted generally by 20 in Figure 1.

A step involving suction of an air flow through an entry section (gate 11d) is initially envisaged, said section being able to remove the whole or part of the air from the confined environment as explained below.

The air flow sucked through a special motorized blower unit constitutes the air flow to be sanitized and therefore initially has a certain degree of contamination owing to the presence of suspended particles.

Below the term "particle" is understood as meaning any contaminating corpuscle or any bacterial or microbial presence in general which constitutes a contaminating element of the air flow.

This air flow is then subjected to an electrostatic filtration step by means of positive polarity corona discharge. During this step the production of an electric field and the ionization of the gases which form the air flow for corona discharge are assisted by means of a special ionizing electrode. The positive ions produced by the ionizing electrode are attracted by a special collector electrode and create, during their migration, an ion cloud which positively charges the suspended particles, determining the subsequent collection thereof on the collector electrode having a polarity opposite to the charge of the particles or simply an earth potential.

Electrostatic filtration is based on the electrostatic force which is applied directly to the particles and envisages beforehand electrostatic charging of the said particles, their collection on an electrode and then their removal upon conferring an electric charge to particles suspended in the air and the capture of said particles charged electrically by means of an electric field.

In greater detail, an ionizing electrode is connected to a high-voltage positive-polarity power supply, while another collector electrode is preferably connected to earth.

The particles are electrically charged by means of the positive ions produced by the corona discharge developed by a suitably charged ionizing electrode. The positively charged particles are then captured owing to the forces of attraction exerted by the collector electrode which is provided with a potential having a sign opposite to that of the said particles. The force of attraction on the particles depends on the intensity of the electric field and on the distance of the said particles from the collector electrode. The captured particles are retained by mechanical, electric and molecular forces.

More particularly a first section of the filter is intended to charge the particles and a second downstream section is intended to collect said particles. For this purpose, it.is envisaged using in this latter section the collector electrode inside a capturing system consist of capturing surfaces or capturing surfaces facing repelling surfaces having a geometry which is variable depending on the circumstances and arranged close together so as to intercept the air flow and together therewith the electrically charged particles.

In accordance with a characteristic feature of the present invention, discharging by means of the positive corona effect is due to charging of the ionizing electrode, which has an elongate shape or other shape useful for generating a localized field with an intensity sufficient for producing the corona effect, with positive polarity.

In these conditions a cloud of positively charged ions is created and is directed towards the other collector electrode which is negatively charged or, preferably, connected to earth.

By controlling the voltage on the positive electrode and/or by modifying the characteristics of the positively charged ion gas the electrostatic filtration process may also be optimized according to the nature of the dimensions of the particles to be removed.

The method then envisages subjecting the air flow to an ozonization step by means of which a controlled concentration of ozone is generated.

According to the invention this concentration of ozone is associated with the positive corona discharge produced during the electrostatic filtration step. By controlling the voltage of the electrodes it is also possible to control effectively the production of ozone within the air flow, which is produced as a result of phenomena associated with the positive corona effect.

Experimentally it has been possible to establish that by means of positive polarization of the electrode responsible for the corona effect it is possible to achieve and control more easily the necessary concentration of ozone (low concentration) than charging the said electrode with a negative charge.

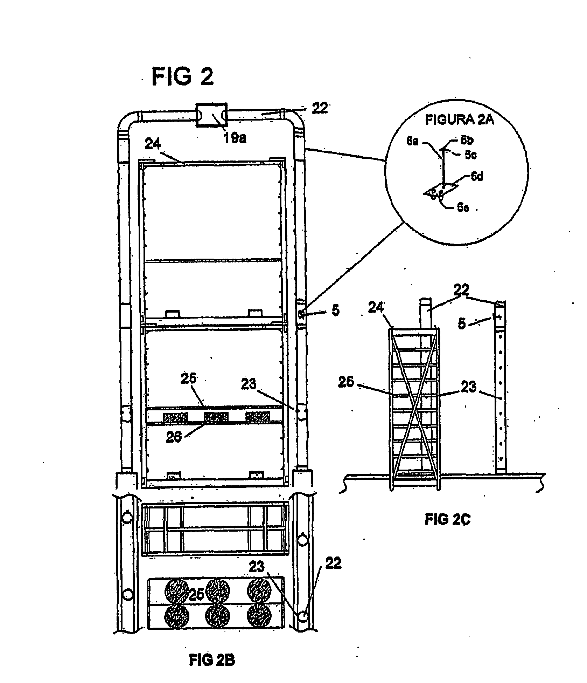

At this point, before passing through any thermo-hygrometric treatment section, consisting of a cooling set and a post-heating set, the air flow is subjected to a step involving generation of a controlled concentration of negatively charged ions (referred to below as "negative ionization") by using ionization means indicated by 5 in Figure 2.

At least one of the ionization means must be placed immediately at the outlet of the positive electrostatic filter. These means intercept the air flow downstream of the electrostatic field so as to create by means of the concentration of negatively charged ions and the concentration of ozone a mixture with a marked microbicidal action able to sanitize the air flow and therefore the surfaces with which it may come into contact.

The final step of conveying the flow thus treated has the purpose of transporting the said flow into the confined environment which is desired and which, as mentioned above and as illustrated in the drawings below, may consist both of an environment for persons and of a compartment of a machine or the like for storing products, in particular of the alimentary kind.

The method of the invention is based on the synergic effect produced by the combination of two principles which are associated together with an advantageous original result. These principles consist of electrostatic filtration and the action of the microbicidal mixture composed of regulated concentrations of negative ions and ozone.

By using electrostatic filtration with a positive electrostatic field it is possible to achieve in an advantageous and original manner a controlled production of ozone necessary for the microbicidal mixture. Moreover, any particles which emerge from the electrostatic filter, retaining their positive charge, are the most exposed to the action of the microbic mixture which is negatively charged owing to the presence of the negative ions.

In greater detail, this circumstance is particularly significant for ensuring a further reduction in the microbic contamination. This is due to the fact that the positively charged particles and the free ions with a positive charge which are not captured during the electrostatic filtration step generate together with the negative ions of the ionization step in particular compound acids with electrochemical reactions able to combat effectively the contamination - in particular bacterial contamination - without thereby significantly damaging the air treatment device.

In fact, in accordance with a characteristic feature of the present invention, the positive ionization due to the electrostatic filtration produces, from the molecular components of air N₂ and O₂, new ionic compounds such as, for example, the ion N⁺, NO⁺, NO₂⁺, O⁺. Some of these ions are fixed onto the surface of the particles contained in the air flow which, in most cases, is captured by the collector electrode of the filter. The filter has an average efficiency of about 99.5% for particles with a size ≥ 0.5 µm. The positively charged particles which are not captured (and which may be partially formed by bacteria) immediately come into contact with negative ions of the type O₂⁻, OH⁻, O₃⁻, H₂O⁻, CO₄⁻ which are produced during the ionization step, which negative ions react with the positive ions, producing neutral molecules (oxides) which in turn, in the presence of humidity, generate molecules of acid substances (oxyacids). The acids and the oxyacids possess a well-known bactericidal effect (for example: NO₂, HNO₃). In this way substances with an anti-bacterial activity and acidity conditions which are hostile to the survival of the bacteria are produced on the surface of the particles.

This effect is obtained by means of the sequence positive electrostatic filtration, negative ionization and air-conditioning able to control the temperature and the relative humidity of the air flow, in particular downstream of electrostatic filtration and ionization.

The anti-bacterial effect of the abovementioned molecules also acts on the heat exchange surfaces where condensate is formed, said condensate being acidified by them.

Advantageously, the air-conditioning step is performed downstream of the filtration and ionization steps so as to allow the air flow to retain the optimum thermo-hygrometric characteristics, and in particular humidity characteristics, for formation of the abovementioned compounds.

Electrostatic filtration removes the most significant part of the microbiological or particle contamination contained in the air flow, managing to eliminate even up to 95-99.5% of the abovementioned contamination.

Owing to the fact that electrostatic filtration is performed upstream of negative ionization it is possible to obtain greater efficiency in the ionization step. In fact, the deposition of biofilm is prevented in the region of the abovementioned ionization means, which biofilm would on the one hand contribute to diffusion of the microbic presence responsible for the contamination and on the other hand would result in a reduction in the efficiency of the said ionization means.

The use of the electrostatic filter combined with negative ionization of the air therefore has the particular advantage of eliminating from the air, in the following order of efficiency: fungal spores, bacteria and particles, with an overall efficiency of 99.5% for particles with a size of ≥ 0.5 µm. The sole negative ionization of the air, on the other hand, would result in a significant deposition of negatively charged particles on the surfaces of the ionization means which are acted on by the flow and in particular on the surfaces which are at zero potential. This deposition would take place in the form of fine or coarse particles or in the form of the fungal spores present in the air in considerable quantities. The sole negative ionization of the air, even in the presence of a synergic quantity of ozone, has a less pronounced effect on the fungal spores than on bacterial vegetative forms. Therefore if a considerable quantity of the latter were to be captured on the surfaces affected by negative ionization, they would probably be most likely to survive and germinate. The biofilm which could form would form a barrier preventing the bactericidal effect of the negative ion - ozone synergic mixture. With the passing of time, therefore, the microbicidal effect could decline and colonies of mould could develop.

It must also be commented that, by means of electrostatic filtration, mainly the particles having dimensions greater than 0.5 µm are retained and this allows a greater efficiency in reduction of the microbic presence in the following air ionization station where it is possible to treat selectively and mainly particles having a size smaller than this value of 0.5 µm.

By means of the action of the microbicidal gaseous mixture composed of negative ions and low concentration ozone, it is possible to eliminate practically entirely the remaining percentage of microbiological or particle contamination contained in the air flow.

In order to combine synergically these two principles, the first step of the method consists in sucking in the air to be treated, from the external environment or from the environment to be sanitized or from the space where the products or surfaces to be decontaminated are situated. The air may vary between air which is fully recirculated, in a closed circuit between the treatment device and the confined environment to be treated, and air which consists entirely of external air, according to requirements. The mixing together of recirculated air and external air allows the adjustment of the chemical and physical parameters of the treated air. After suction, the next step consists in suitable filtration of the sucked-in air in order to remove the most significant part of the microbiological and particle contamination which it contains. This filtration may also be performed using mechanical filters in combination with the abovementioned electrostatic filters. The mechanical and/or electrostatic filters, which may be of various types, are arranged in sequence so as to achieve an increasing level of filtration efficiency, from initial coarse filtration, which removes the visible impurities, to absolute filtration, as defined in the standard EN 1822 of the CEN. The number of filters and their type as well as the number of filtration stages depends on the contamination category which is to be achieved in the environment to be treated in the case of environmental sanitization or the programmed residual contamination on the surfaces and in the spaces to be treated, in the case of localized application. After electrostatic filtration, combined if necessary with mechanical filtration or with opposite-polarity electrostatic filtration, the filtered air cleaned of most of the contaminants contained therein undergoes an immediate negative ionization treatment which causes electrochemical reactions with the positive ions, producing new chemical molecules or compounds with an anti-bacterial action and, following adjustment of the thermo-hygrometric parameters, then undergoes the sanitization process by means of the microbicidal gaseous mixture comprising negative ions and low concentration ozone. This mixture is produced directly in the air, in a continuous or intermittent manner, with the simultaneous production of both components of the mixture or individually in each case, in a constant manner or with the possibility of modulating the concentration of both the components of the mixture. In particular, the concentration of negative ions (number of ions per unit of volume) must be significantly greater than the concentration present under natural conditions.

In particular the concentration of negative ions within the microbicidal mixture is at least 50,000 ions per cubic centimetre. As regards the ozone, which is emitted in low concentrations in the microbicidal gaseous mixture, continuously or intermittently, its presence and its concentration are regulated according to the type of application and take into account the TLV (Threshold Limit Value) for ozone adopted by the ACGIH (American Conference of Governmental Industrial Hygienists) and subsequently by the AIDII (Italian Association of Industrial Hygienists) and the existing regulations. The value for the ozone concentration within the air flow must therefore preferably not exceed values compatible with the value of TLV equal to 0.1 ppm for the zones occupied by persons.

The concentrations of ozone and negative ions used are suitably determined on the basis of the specific application, where it is necessary to take into account the presence of persons and/or animals, the type of materials and/or products which are exposed to treatment and to the desired level of contamination. Therefore the concentrations of ozone and negative ions in the air and their relative ratio are automatically determined, regulated and controlled. In the case where the fluid is introduced into the working environment, in contact with living animals, or directly into the atmosphere, the concentrations of negative ions and ozone are reduced to within the levels permitted by the existing regulations. In localized applications, the concentrations may be greater so as to obtain a greater microbicidal effect on the surfaces which are acted on by the treated air flow. In many applications, the controlled concentration of ozone remains within the limits laid down by the specific regulations, reducing where necessary the concentration reducing action to the negative air ions alone. The flexibility in metering of the concentrations of the components of the microbicidal gaseous mixture is an important feature for the devices which apply the method, in particular in the case of localized applications, where the microorganisms may change from one product to another and have resistances which differ from one type of microorganism to another. The lethal levels, microbiologically speaking, of the concentrations of the components of the microbicidal mixture will be maintained wherever necessary in the devices which apply the method.

Following filtration and the first sanitization stage, the filtered and sanitized air may be treated from the thermo-hygrometric point of view and then treated again by a final sanitization stage. The last step of the method differs depending on its aims: environmental sanitization or localized sanitization. In the case of environmental sanitization, this last step consists in reducing the positive and/or negative ions of the air and the low concentration ozone before introduction of the treated air into a confined environment. Owing to this reduction it is possible to maintain in the confined environments which are subjected to the method a sanitized and purified atmosphere in accordance with the existing regulations which govern the quality of the air in public and/or working environments. If necessary it is possible to envisage restoring a suitable concentration of negative air ions similar to that which can be found in nature. In the case of localized sanitization, the concentration of the negative ions in the air and/or low concentration ozone is incremented locally, for localized application by means of a distribution or diffusion system, directly onto the surfaces and into the spaces to be treated. The last step in the method may be combined with other treatments which are known per se, in order to remove from the air other undesirable volatile or gaseous substances which pollute the environments to be sanitized.

The difference between the devices intended for environmental sanitization and those used for localized sanitization consists in the application of the method mainly to the air in order to obtain an aseptic or controlled contamination atmosphere within confined environments in the first case as opposed to using the air mixed with the microbicidal gaseous mixture as a sanitizing fluid for decontaminating surfaces and products in localized applications, in the second case. The device which implements the method according to the invention for environmental sanitization may have numerous applications in air conditioning and treatment plants in general, in systems for controlling the contamination of premises which are sterile or classified as sterile rooms and in environments where there are aseptic or low contamination operating conditions as in the case of the processing, packaging and bottling of perishable products and food products which do not have preservatives or which are subject to minimum preservation measures. With reference to the accompanying illustrative plates, Figure 1 shows the diagram for the design of a preferred but not exclusive embodiment of a device for environmental sanitization, illustrated by way of a non-limiting example, for implementing the method of the invention. The device, which is generally denoted by the reference number 21, comprises an air treatment unit 20, which is connected by means of a delivery conveying duct 19a and return conveying duct 19b in a normally closed circuit to a confined environment 10 to be treated, with or without the incorporation of air supplied from the outside 19c. For the purposes of the present invention the term "air treatment unit", over and above the example in question, must be regarded as referring to any apparatus, such as, for example, an air treatment plant which is designed to house the device according to the invention or is capable of operating using the method according to the present invention. The air circulates within this circuit, which is normally closed, always in the same direction indicated in the diagram of Figure 1 by an arrow. The air treatment unit 20 is provided with the possibility of incorporating external air via a suitably calibrated input gate 11d which is regulated together with the control gate 11b which calibrates the reintroduction of the recirculating air flow depending on the application, the environmental parameters or the sanitized processing cycle. The regulation of the gates 11b and 11d may be permanently fixed or adapted to the circumstances, with manual or motor-driven gates. The expulsion gate 11c, which is arranged on the expulsion duct 19c, is normally closed, so as to allow recirculation. Depending on the circumstances, an additional extractor fan (not shown) may or may not be used in order to allow efficient expulsion, being situated inside the duct 19c, ahead of the eliminating device 9 and the gate 11c. The expulsion gate 11c allows the evacuation of the recirculating air if it is required to replace the atmosphere of the confined environment 10 with fresh outside air. In this case the gates 11c and 11d are both opened, while the gate 11b is simultaneously closed. The air treatment unit 20 is composed of a mixing chamber 19d for mixing the recirculating environmental air and the external air, followed by a mechanical pre-filter 1 for coarse dust. After pre-filtration, a motorized blowing unit 2 for ventilation and forced circulation of the air is provided. This unit generally consists of a fan known per se and designed to create the air flow in the device. In the preferred embodiment, the appliance 2 is located before the filtering device 4, namely on the delivery side with respect to the filters 4 so as to allow filtration also of the particles emitted by the moving parts within the air flow which, after the filters, acts on the heat exchanger sets 7a (for cooling and dehumidification) and 7b (for heating). This solution also tends to avoid any sucking-in of air which has not been filtered by the access elements of the filtering device 4 and eliminate the dust generated by the operation of the motor of the fan 2. Alternatively, the fan 2 may be positioned after the filtering section 4, but this arrangement is considered to be less reliable from the hygiene point of view. The air flow produced by the appliance 2 is turbulent and is converted into a uniform flow by means of the structure 12 which is generally formed by a simple metal plate which is perforated uniformly over at least 60% of its surface, before passing into the main filtration section 4. Primary filtration 4 is performed by electrostatic filters of the type explained above and has the object of eliminating most of the contaminants present in the air, both of an inorganic and of an organic nature, including those microorganisms which are killed on the surface of the electrostatic filter, which moreover ensures a limited loss of load. Advantageously, the electrostatic filter may also be combined with other types of filters. In fact, the combination of various types of filters allows higher levels of filtration to be achieved, up to the absolute highest filtration defined for filters of the type known as HEPA (High Efficiency Particulate Air Filters) and ULPA (Ultra Low Penetration Air Filters). After filtration 4, the air flow passes into a section where air ionization means 5 are provided, said means by generating negative ions triggering an ion reaction with the positive ions which are free or attached to the particles emitted from the filter, resulting in substances with an anti-bacterial activity to which the synergic mixture of negative ions and ozone also contributes. The following air ionization means 5, which are situated after the thermo-hygrometric treatment device, together with the ozone produced by the electrostatic filter, again produce a microbicidal gaseous mixture consisting of a gaseous mixture of negative air ions and low concentration ozone. This microbicidal gaseous mixture is mixed thoroughly with the recirculating air flow, sterilizing it. It is possible to envisage the additional use of ozonization means 6 for producing ozone in addition to that produced by the electrostatic filter. The ionization means 5 forms part of an appliance composed of at least one ionization means, which produces negative ions in the air, and a high-voltage power supply system (not shown). The ionization means 5 of the appliance may be provided with voltage regulators 18 which allow a metered supply of the concentration of negative ions emitted. Similarly, the additional and optional ozonization device 6 forms part of an appliance which comprises at least one unit generating low concentration ozone, and a high-voltage direct current power supply system which is different from or combined with that for ionization of the air (not shown). Both the ionization means and the ozonizer are preferably positioned centrally with respect to the air flow conveying cross-section, with the tip of the ionization electrode of the ionization means 5 directed in the direction of the air flow. A preferred, but not exclusive or limiting embodiment for the ionization means is shown in the detailed view 2a of the drawings in Figure 2 and is described hereinbelow in the first device for localized application of the method. The ionization means and additional ozonizer both base their operation, generally but not exclusively, on the known corona effect, and require high-voltage power supplies which may operate at different potentials and are connected to the emission devices (ionization means and ozonizers by means of conventional electrical connections (not shown). The ionization of the air is performed in a continuous, constant or intermittent manner, as is also the production of ozone which will be kept at suitable levels of concentration for the environments to be sanitized, by providing apparatus identical to 5 and 6 along the whole path of the ducts 19a and 19b. The number of air ionization means and ozonization units is determined by the concentration levels to be achieved, by the volumes of air to be treated and by the length of the ducts 19a and 19b. The ionized gaseous mixtures generated in the air, in combination with the ozone, exert the marked anti-microbic action described above in the present description, within the entire recirculating air flow, as well as on the surfaces which come into contact with the microbicidal gaseous mixture produced, in the case of all the components which form the air treatment unit 20 and the conveying ducts 19a and 19b, including the appliances and the apparatus which they contain. After the initial introduction of the microbicidal gaseous mixture into filtered air flow it may be required to perform conditioning of the sanitized air, namely setting of its temperature, with or without control of its humidity. For performing conditioning an air-conditioning apparatus which is known per se will be provided, said unit consisting generally, but not exclusively, of finned exchanger sets complete with regulating systems (also known). In the particular (non-limiting) case of the device illustrated in the diagram of Figure 1, a cooling exchanger set 7a and a heating exchanger set 7b have been provided in order to allow control of the humidity. The choice as the type of exchanger, their dimensions and the possible combinations (exclusively for cooling or alternately exclusively for heating or, as shown in the diagram of Figure 1, paired for cooling and heating, etc.), the constructional details, the system for regulating operation thereof, the choice of fluids which power the sets and the need to provide defrosting systems and systems for collecting the condensation or defrosting water, are based on a series of known arrangements and appliances associated with air-conditioning and not illustrated in the present description. At the same time, regulation of the temperature and the humidity in the confined environment 10 is performed by means of an independent circuit with special valves, probes and electronic controls which are not shown in the diagram of Figure 1. In the preferred but not exclusive embodiment shown in Figure 1, the heat exchanger sets can be isolated from the rest of the air treatment unit by means of an exclusion gate 11a, which allows isolation of the live parts during the operations for extraordinary maintenance of the exchangers. Moreover, during these operations, an alternative and temporary decontamination system 3, using a UV lamp or the like and arranged upstream of the main filtering section 4, is activated, since during the maintenance operations the microbicidal gaseous mixture cannot be introduced into the air, the fans being at a standstill. The introduction of sanitized and purified air from the duct 19a into the confined environment 10 and return into the section 19b are performed respectively by means of the conventional diffusers 16 and the return grills 17 of the type known per se. In the case where the air is introduced into the confined environment 10, in contact with persons or animals, or directly expelled into the atmosphere, it may be indispensable to reduce the concentrations positive ions and/or negative ions and/or ozone to within the limits permitted by the existing regulations. This may occur when, owing to operational choices on the part of the user, the concentrations of the components of the microbicidal mixture within the sections 20, 19a and 19b of the device are higher than those permitted by the existing environmental air regulations or when the periods of contact exceed the time limits stipulated in these regulations. In this case, prior to introduction via the diffusers 16 or expulsion by means of the gate 11c, the sanitized air is treated so as to neutralize the positive and/or negative ions and/or the ozone. The neutralization of the positive and/or negative ions of the air is performed by means of eliminating means consisting for example of a polarized grid 8 having a polarity opposite to that of the air ions, while neutralization of the low-concentration ozone is performed using eliminating means consisting for example of the appliance 9 which may be an active-carbon filter or any other type of appliance suitable for eliminating low concentrations of ozone. In view of the short life span of the air ions, it is not necessary to envisage a neutralization means 8 along the expulsion duct. The presence of the apparatus 8 and 9 is not systematic, but depends on the type of environmental sanitization to be performed. The last treatment carried out on the sanitized air consists in optimization of the air by means of restoration of the natural concentration of negative ions generated by the ionization means 14 before introduction into the confined environment 10. The different concentrations of the ozone are detected in the various parts of the device 21 and in the confined environment 10 by special concentration sensors 13 specifically calibrated for detecting low concentration of ozone and connected to a regulator 15.

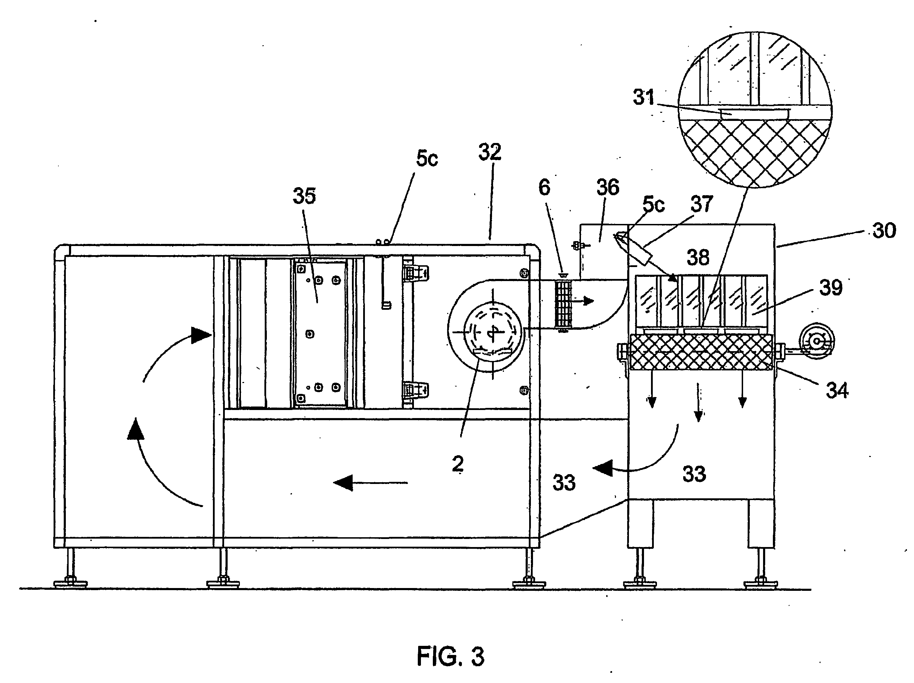

The device which implements the method for localized sanitization may have numerous applications in systems and machines which use air as a treated or process fluid, systems for controlling the contamination of sterile rooms and booths, granulators, driers, degassers, pneumatic conveyors, saturators and systems for controlling the contamination of packaging and bottling apparatus. With reference to the accompanying illustrative plates, Figures 2 and 3 show examples of embodiment of a preferred, but not exclusive embodiment of two devices for localized sanitization, which are illustrated by way of a non-limiting example and implement the method according to the invention. More specifically, Figure 2 shows a device for localized application of the method, for sanitization of curing warehouses. The device for the production of sanitized air is similar to that described in the diagram according to Figure 1, but the flow of ionized and ozonized air conveyed inside the delivery ducts 19a, instead of being introduced into the environment 10 by means of diffusers, is sent into a capillary distribution system consisting of pipes or ducts 22 situated in the middle of the products to be cured. These ducts are provided with calibrated nozzles 23 which are made of metal or conductive synthetic material and which diffuse locally the purified, sanitizing, ionized and ozonized air with low concentration ozone directly into the environment surrounding the product, on the surfaces of which it exerts a microbicidal action. By way of example, movable storage systems 24 are shown, but the application is also suitable for fixed systems. The air distribution system is also known and was used for the first time in Holland in the 1980's. The air treated by means of electrostatic filtration and then enriched with a measured concentration of ozone is suitably supplied with a controlled concentration of negative ions of the said air in order to produce the microbicidal gaseous mixture, by means of ionization means 5 which are identical to these used in the main conveying ducts and in the air treatment unit of the device for sanitization of the ambient air (see Figure 1). An example a preferred, but not exclusive embodiment of ionization means 5 for positioning in a duct is illustrated by way of non-limiting example in a detailed view 2a of Figure 2. The appliance consists of at least one emission electrode 5a in the form of a needle, which is made of a special alloy, with a suitable surface treatment, and is connected to a power supply contact 5b which also allows a jumper connection with the other electrodes by means of a conventional high-voltage wire and is supported by a spacing rod 5a which is provided with isolators and allows the electrode to be positioned in the centre of the duct cross-section. The rod is mounted on a support plate 5d where the connections for the external high-voltage wires 5a are also located, i.e. a connection for the power supply and a connection for the jumper wire. Ionization means 5 are inserted every two or three metres inside the pipes which are designed with suitable dimensions for ensuring an adequate speed of the air. The purified, sanitized and sanitizing air is diffused locally in such a way as to allow the calibrated nozzles 23 to expel the air between the product 26 and the upper support surface 25. If, this surface does not exist, because the product is hung, the calibrated nozzles are positioned between the products arranged underneath and those arranged above. Expulsion of the sanitizing air occurs at an output speed from the calibrated nozzles, which is appropriate for the type of product and the storage system. The product under these conditions is surrounded by a germicidal atmosphere which prevents locally the growth of undesirable microorganisms on the products being cured.

Figure 3 shows a second example of localized sanitization where the term "localized" is applied in an even narrower sense, referring to the application of the device no longer within a room as in the example above but on process machines. Figure 3 shows, for example, the front and side views of a transfer tunnel 30 intended to convey containers 31 (jars, small trays, loose material, etc.) intended for the packaging of fresh food products to a filling machine for these products (not shown). The main components of the sanitization device, indicated overall as the device 32, are identical to those of the air treatment unit 20 illustrated in Figure 1. They are merely designed with different dimensions and shaped so as to allow assembly on the process machine in question. The sanitization device 32 is composed of a fan 2, which recirculates the sanitization air, sucking it from the intake manifold 33, which is situated underneath the meshed conveyor belt 34 of the transfer tunnel 30. After passing through the electrostatic filter 35, the filtered air containing the concentration of ozone may also pass through an additional and optional ozonization unit 6 able to regulate further the quantity of ozone before it is supplied into the distribution system consisting of the manifold 36 and the nozzles 37, which contain the air ionization electrodes 5c. These nozzles direct onto the meshed conveyor belt 34 and onto the product to be sanitized (containers 31) a microbicidal gaseous mixture with a particularly high concentration of positive and/or negative air ions, thus obtaining a more effective and more rapid sanitizing effect. The flow of sanitizing air constantly maintains a slight overpressure inside the entire compartment 38 and prevents the air of the surrounding environment from penetrating into the machine. The compartment is bounded by a strip-like curtain 39 situated at the belt inlet and outlet and made of synthetic material which is conductive or rendered conductive so as to allow neutralization of the ions in the microbicidal gaseous mixture.

With regard to the object of sanitizing the air and the production of sanitizing air, the advantages of the invention are as follows:
- the creation of a method and device which allows the prevention, elimination and removal in a very effective manner of all contaminating bacteria, contaminating mould, yeasts, bacterial and fungal spores and microorganisms in general, as well as toxins produced by the metabolism and/or deterioration of these microorganisms, present in the air and present on the surfaces in contact with the treated air;
- the creation of a method and device which perform the task of sanitization referred to above in the following manner and with the following features:

- using active sanitization, i.e. not merely mechanical removal;
- with a permanent sanitizing effect and continuous or intermittent operation of the sanitization devices;
- with the use of a filtration device associated with a device for the production of ions so as to form a mixture composed of negative air ions and low concentration ozone in order to make use of the increased germicidal properties of these two components due to their synergy following mixing thereof, thus allowing the introduction of reduced concentrations of these components into the air to be sanitized (environmental application) or into the sanitizing air (localized application). The attainment of this object is of great importance for guaranteeing and protecting the working conditions and health of the operating personnel. Both the concentration of positive and/or negative air ions and the concentration of ozone may be pre-set and controlled;
- the possibility of installing the sanitization device directly onto machines, process lines and/or appliances which use processing air or treat it;
- the possibility of using the invention for the production of sterile atmospheres, purified atmospheres, controlled-contamination atmospheres for sterile rooms, aseptic packaging atmospheres and modified atmospheres, or for sanitization of the air and environment for premises intended for sanitary use, working premises, curing, storage warehouses, refrigerated warehouses and livestock rearing farms; or for sanitization of the air in confined environments for machines which prepare, convert, process and package sterile or controlled contamination products for the following industrial sectors (non-limiting and non-exhaustive list): agricultural/alimentary sector, cosmetics sector, pharmaceutical sector, chemical sector, hospital and sanitary sector, electronics sector, sanitary product and components sector, packaging materials sector and space industry and military sector;
- the possibility of using the invention to prevent the diffusion of mites associated with the proliferation of other microorganisms, such as moulds present in certain agricultural/alimentary processes;
- the possibility of using the invention in livestock rearing farms in order to prevent the spread of animal diseases.

The characteristic features and advantages as well as other advantages not listed have a particularly important implication for the sectors listed above. In the food sector in particular, as a result of the invention it is possible to prolong the shelf life of perishable products, avoid the contamination of environments and products which are not perishable, but which must have optimum aseptic qualities, such as containers, and increase the healthy character of fresh products and food products which may only be subject to limited conservation and preservation measures. The attainment of these objectives results in an increase in the safety of food products for the consumer in general.

The same Applicant has already carried out tests in order to evaluate the effects of the system, introducing into an experimental unit considerable quantities of microorganisms and spores and assessing the reduction in the microbic content after a predefined period under controlled conditions (Challenge Test). It has thus been possible to establish how the method and the device described achieve the aim and the predefined objects since they allow a reduction in the contamination present in the air and on the surfaces acted on by the fluid itself. The method and the device according to the invention may be subject to numerous variations and modifications, all falling within the scope of the same inventive idea expressed here. Moreover, all the details may be replaced by other technically equivalent elements. In practice the materials used, as well as the dimensions and the contingent forms, may be of any nature according to requirements.

## Claims

1. Method for the environmental or localized sanitization of air, by means of a treatment unit, comprising:
- a step involving suction of an air flow with suspended particles inside said unit;
- a step involving electrostatic filtration of said flow, by means of:
• production of an electric field with at least one positively charged ionizing electrode able to ionize by means of a positive corona discharge said air flow with the generation of positive ions and able to generate by means of said positive corona discharge a controlled concentration of ozone within said flow, involving an ozonization step during said electrostatic filtration step;
• electric charging of said suspended particles substantially with a positive charge by means of said positive ions;
• collection of said positively charged suspended particles on at least one collector electrode having a charge opposite to that of said ionizing electrode, or with an earth potential;
- an ionization step for generating a concentration of negatively charged ions in said air flow by means of ionization means able to process said flow downstream of said electrostatic filtration and ozonization steps, said concentration of negatively charged ions and said concentration of ozone forming together a mixture with a marked microbicidal action for sanitizing said flow and/or the surfaces with which it may come into contact;
- a step involving conveying of said flow thus treated from said unit to a confined environment.

2. Method according to Claim 1, **characterized in that** the concentration of ozone within said air flow does not exceed the TLV (Threshold Limit Value), in particular of 0.1 ppm in an environment occupied by persons, calculated on the basis of eight working hours per day and forty hours per week.

3. Method according to any one of the preceding claims, **characterized in that** it comprises at least one step involving air-conditioning of said air flow, said air-conditioning step being carried out on said air flow after said filtration step and ionization step and being able to regulate the thermo-hygrometric characteristics of said flow to be introduced into said confined environment during said conveying step.

4. Method according to any one of the preceding claims, **characterized in that** said positive ions or said particles positively charged by said positive ions, whichare not captured during said electrostatic filtration step, produce oxide compounds reacting with the ions generated during said negative ionization step, and in presence of humidity said oxide compounds produce in turn acid compounds, capable of reducing the contamination, in particular bacterial contamination.

5. Method according to Claim 3 and 4, **characterized in that** said air-conditioning step performed downstream of said filtration and ionization steps allows said air flow to retain thermo-hygrometric and in particular humidity characteristics able to allow the formation of said compounds.

6. Method according to any one of the preceding claims, **characterized in that** it comprises at least one step involving reduction of the positive and/or negative ions of said air flow, said step being carried out on said air flow after said electrostatic filtration, ionization, ozonization and conditioning steps and before said step involving conveying into the confined environment.

7. Method according to Claim 1, **characterized in that** it comprises at least one step involving a reduction in the concentration of the ozone; said ozone reduction step being carried out on said air flow after said electrostatic filtration, ionization, ozonization and conditioning steps and before said step involving conveying into the confined environment.

8. Method according to Claim 1, **characterized in that** it comprises at least one step involving restoration of the natural level of concentration of the ions present in the air, said step being carried out on said air flow after said step involving a reduction in the positive and/or negative ions of the air and reduction of the ozone.

9. Method according to Claim 1, **characterized in that** it comprises at least one step involving mixing of said air flow treated with a flow of air drawn from said confined environment and/or with a flow of air outside said confined environment.

10. Method according to one or more of the preceding claims, **characterized in that** said conveying step is intended to distribute said treated air flow over surfaces able to be sanitized.

11. Method according to one or more of the preceding claims, **characterized in that** said ozonization step is performed partially by means of ozonization means arranged downstream of said filtration means and in the vicinity of said ionization means for generation of said microbicidal mixture.

12. Method according to one or more of the preceding claims, **characterized in that** said step involving generation of a concentration of negatively charged ions and said ozonization step for the generation of a controlled concentration of ozone are obtained by acting directly on the gaseous components which compose said air flow.

13. Method according to Claim 1, **characterized in that** said controlled concentration of negative ions within said microbicidal mixture is equal to at least 50,000 ions per cubic centimetre.

14. Method according to Claim 1, **characterized in that** said step involving generation of a concentration of negatively charged ions is performed by means of continuous or intermittent or constant or modulated ionization of said air flow and **in that** correspondingly said step involving generation of a controlled concentration of ozone is respectively continuous or intermittent or constant or also modulated so as to form a microbicidal mixture with predefined concentration ratios of negative ions and ozone.

15. Method according to Claim 1, **characterized in that** said localized sanitization is performed solely on given surfaces, in particular products or loose materials.

16. Method according to Claim 6, **characterized in that** said confined environment consists of an environment suitable for receiving persons and **in that** said reduction step is intended to reduce the concentrations of said positive and/or negative ions in said air flow to values which are commonly found naturally in the atmosphere.

17. Method according to Claim 7, **characterized in that** said confined environment consists of an environment able to receive persons and **in that** said reduction step is intended to reduce the concentrations of ozone to predetermined values and in particular to reduce the concentration of O₃ to 200 micrograms per cubic metre, by way of average concentration per hour, and 65 micrograms per cubic metre, by way of average concentration over twenty four hours.

18. Device for the environmental or localized sanitization of air, in particular for implementing the method according to one or more of the preceding claims, which is able to be operationally associated with a treatment unit, **characterized in that** it comprises:
- means for the forced circulation of an air flow with suspended particles, between at least one intake section and at least one delivery section of said treatment unit;
- means for electrostatic filtration of said flow, by means of production of a controlled electric field and positive corona discharge produced by means of at least one ionizing electrode with positive polarity capable of generating positive ions and positively charging said suspended particles, so as to collect them on at least one collector electrode which is positioned downstream of said ionizing electrode with respect to the direction of advancing movement of said air flow, said positive corona discharge being able to generate a controlled concentration of ozone;
- ionization means arranged downstream of said electrostatic filtration means with respect to the direction of advancing movement of said air flow and able to generate a concentration of negatively charged ions within said flow, said concentration of negatively charged ions and said concentration of ozone forming together a mixture with a marked microbicidal action for sanitizing said flow and/or the surfaces with which it may come into contact.

19. Device according to Claim 18, **characterized in that** it comprises monitoring means for detecting the concentration of ozone emitted from said ozonization means.

20. Device according to Claim 18, **characterized in that** it comprises ozone reducing means and positive and/or negative ion reducing means arranged downstream of said ionization means, for controlling the introduction into the environment of the respective concentrations of ozone and negative ions within said air flow.

21. Device according to Claim 18, **characterized in that** said flow output from said delivery section is directed by means of conveying means onto surfaces and products to be sanitized by means of said microbicidal mixture.

22. Device according to Claim 18, **characterized in that** said ionization means comprise electrodes emitting negative ions.

23. Device according to Claim 18, **characterized in that** said electric field is obtained between an ionizing electrode subject to a controlled positive voltage and a collector electrode arranged facing said ionizing electrode and charged with negative polarity or connected to earth.

## Revendications

1. Procédé pour la purification environnementale ou localisée d'air, par l'intermédiaire d'une unité de traitement, comprenant :
- une étape impliquant l'aspiration d'un écoulement d'air avec des particules suspendues à l'intérieur de ladite unité ;
- une étape impliquant la filtration électrostatique dudit écoulement, par l'intermédiaire de :
■ la production d'un champ électrique avec au moins une électrode ionisante chargée positivement pouvant ioniser, par l'intermédiaire d'une décharge positive par effet corona, ledit écoulement d'air avec la production d'ions positifs et pouvant engendrer, par l'intermédiaire de ladite décharge positive par effet corona, une concentration contrôlée d'ozone dans ledit écoulement, impliquant une étape d'ozonisation pendant ladite étape de filtration électrostatique ;
■ chargement électrique desdites particules suspendues sensiblement avec une charge positive par l'intermédiaire desdits ions positifs ;
■ captage desdites particules suspendues chargées positivement sur au moins une électrode collectrice ayant une charge opposée à celle de ladite électrode ionisante, ou avec un potentiel de masse ;
- une étape d'ionisation pour engendrer une concentration d'ions chargés négativement dans ledit écoulement d'air par l'intermédiaire de moyens d'ionisation pouvant traiter ledit écoulement en aval desdites étapes de filtration électrostatique et d'ozonisation, ladite concentration d'ions chargés négativement et ladite concentration d'ozone formant ensemble un mélange avec une action microbicide marquée pour purifier ledit écoulement et/ou les surfaces avec lesquelles il peut venir en contact ;
- une étape impliquant le transport dudit écoulement ainsi traité de ladite unité vers un environnement confiné.

2. Procédé selon la revendication 1,
**caractérisé en ce que** la concentration d'ozone à l'intérieur dudit écoulement d'air n'excède pas la VLT (valeur limite de seuil), en particulier de 0,1 ppm dans un environnement occupé par des personnes, calculée sur la base de huit heures de travail par jour et de quarante heures par semaine.

3. Procédé selon une quelconque des revendications précédentes,
**caractérisé en ce qu'**il comprend au moins une étape impliquant la climatisation dudit écoulement d'air, ladite étape de climatisation étant mise en oeuvre sur ledit écoulement d'air après ladite étape de filtration et ladite étape d'ionisation et pouvant réguler les caractéristiques thermo-hygrométriques dudit écoulement à introduire dans ledit environnement confiné pendant ladite étape de transport.

4. Procédé selon une quelconque des revendications précédentes,
**caractérisé en ce que** lesdits ions positifs ou lesdites particules chargées positivement par lesdits ions positifs, qui ne sont pas capturés pendant ladite étape de filtration électrostatique, produisent des composés oxydés réagissant avec les ions engendrés pendant ladite étape d'ionisation négative, et en présence d'humidité lesdits composés oxydés produisent à leur tour des composés acides, pouvant réduire la contamination, en particulier la contamination bactérienne.

5. Procédé selon la revendication 3 et la revendication 4,
**caractérisé en ce que** ladite étape de climatisation réalisée en aval desdites étapes de filtration et d'ionisation permet audit écoulement d'air de conserver des caractéristiques thermo-hygrométriques et en particulier d'humidité pouvant autoriser la formation desdits composés.

6. Procédé selon une quelconque des revendications précédentes,
**caractérisé en ce qu'**il comprend au moins une étape impliquant la réduction des ions positifs et/ou négatifs dudit écoulement d'air, ladite étape étant mise en oeuvre sur ledit écoulement d'air après lesdites étapes de filtration électrostatique, d'ionisation, d'ozonisation et de climatisation et avant ladite étape impliquant le transport dans l'environnement confiné.

7. Procédé selon la revendication 1,
**caractérisé en ce qu'**il comprend au moins une étape impliquant une réduction de la concentration de l'ozone ; ladite étape de réduction de l'ozone étant mise en oeuvre sur ledit écoulement d'air après lesdites étapes de filtration électrostatique, d'ionisation, d'ozonisation et de climatisation et avant ladite étape impliquant le transport dans l'environnement confiné.

8. Procédé selon la revendication 1,
**caractérisé en ce qu'**il comprend au moins une étape impliquant la restauration du niveau naturel de concentration des ions présents dans l'air, ladite étape étant mise en oeuvre sur ledit écoulement d'air après ladite étape impliquant une réduction des ions positifs et/ou négatifs de l'air et réduction de l'ozone.

9. Procédé selon la revendication 1,
**caractérisé en ce qu'**il comprend au moins une étape impliquant le mélange dudit écoulement d'air traité avec un écoulement d'air aspiré à partir dudit environnement confiné et/ou avec un écoulement d'air à l'extérieur dudit environnement confiné.

10. Procédé selon une ou plusieurs des revendications précédentes,
**caractérisé en ce que** ladite étape de transport est destinée à répartir ledit écoulement d'air traité sur des surfaces pouvant être purifiées.

11. Procédé selon une ou plusieurs des revendications précédentes,
**caractérisé en ce que** ladite étape d'ozonisation est réalisée partiellement par l'intermédiaire de moyens d'ozonisation agencés en aval desdits moyens de filtration et à proximité desdits moyens d'ionisation pour engendrer ledit mélange microbicide.

12. Procédé selon une ou plusieurs des revendications précédentes,
**caractérisé en ce que** ladite étape impliquant la production d'une concentration d'ions chargés négativement et ladite étape d'ozonisation pour la production d'une concentration contrôlée d'ozone sont obtenues en agissant directement sur les composants gazeux qui composent ledit écoulement d'air.

13. Procédé selon la revendication 1,
**caractérisé en ce que** ladite concentration contrôlée d'ions négatifs à l'intérieur dudit mélange microbicide est égale à au moins 50.000 ions par centimètre cube.

14. Procédé selon la revendication 1,
**caractérisé en ce que** ladite étape impliquant la production d'une concentration d'ions chargés négativement est réalisée par l'intermédiaire d'une ionisation continue ou intermittente ou constante ou modulée dudit écoulement d'air et **en ce que** de façon correspondante ladite étape impliquant la production d'une concentration contrôlée d'ozone est respectivement continue ou intermittente ou constante ou également modulée de façon à former un mélange microbicide avec des rapports de concentration prédéfinis d'ions négatifs et d'ozone.

15. Procédé selon la revendication 1,
**caractérisé en ce que** ladite purification localisée est seulement réalisée sur des surfaces données, en particulier des produits ou des matériaux meubles.

16. Procédé selon la revendication 1,
**caractérisé en ce que** ledit environnement confiné est constitué d'un environnement approprié pour recevoir des personnes et **en ce que** ladite étape de réduction est destinée à réduire les concentrations desdits ions positifs et/ou négatifs dans ledit écoulement d'air à des valeurs qui sont communément trouvées naturellement dans l'atmosphère.

17. Procédé selon la revendication 7,
**caractérisé en ce que** ledit environnement confiné est constitué d'un environnement pouvant recevoir des personnes et **en ce que** ladite étape de réduction est destinée à réduire les concentrations d'ozone à des valeurs prédéterminées et en particulier à réduire la concentration de O₃ à 200 microgrammes par mètre cube, à titre de concentration moyenne par heure, et de 65 microgrammes par mètre cube, à titre de concentration moyenne sur vingt-quatre heures.

18. Dispositif pour la purification environnementale ou localisée d'air, en particulier pour mettre en oeuvre le procédé selon une ou plusieurs des revendications précédentes, qui peut être associé de façon fonctionnelle à une unité de traitement,
**caractérisé en ce qu'**il comprend :
- des moyens pour la circulation forcée d'un écoulement d'air avec des particules suspendues, entre au moins une section d'admission et au moins une section d'évacuation de ladite unité de traitement ;
- des moyens pour la filtration électrostatique dudit écoulement, par l'intermédiaire de production d'un champ électrique commandé et décharge à effet corona positive produit par l'intermédiaire d'au moins une électrode ionisante avec polarité positive pouvant engendrer des ions positifs et de charger de façon positive lesdites particules suspendues, de façon à les capter sur au moins une électrode collectrice qui est positionnée en aval de ladite électrode ionisante par rapport à la direction du mouvement d'avance dudit écoulement d'air, ladite décharge à effet corona positive pouvant engendrer une concentration contrôlée d'ozone ;
- des moyens d'ionisation agencés en aval desdits moyens de filtration électrostatique par rapport à la direction du mouvement d'avance dudit écoulement d'air et pouvant engendrer une concentration d'ions chargés négativement à l'intérieur dudit écoulement, ladite concentration d'ions chargés négativement et ladite concentration d'ozone formant ensemble un mélange ayant une action microbicide marquée pour la purification dudit écoulement et/ou des surfaces avec lesquelles il peut venir en contact.

19. Dispositif selon la revendication 18,
**caractérisé en ce qu'**il comprend des moyens de contrôle pour détecter la concentration d'ozone émise à partir desdits moyens d'ozonisation.

20. Dispositif selon la revendication 18,
**caractérisé en ce qu'**il comprend des moyens de réduction d'ozone et des moyens réduisant les ions positifs et/ou négatifs agencés en aval desdits moyens d'ionisation, pour commander l'introduction dans l'environnement des concentrations respectives d'ozone et d'ions négatifs dans ledit écoulement d'air.

21. Dispositif selon la revendication 18,
**caractérisé en ce que** ledit écoulement sorti de ladite section d'évacuation est dirigé par l'intermédiaire de moyens de transport sur des surfaces et des produits à purifier par l'intermédiaire dudit mélange microbicide.

22. Dispositif selon la revendication 18,
**caractérisé en ce que** lesdits moyens d'ionisation comprennent des électrodes émettant des ions négatifs.

23. Dispositif selon la revendication 18,
**caractérisé en ce que** ledit champ électrique est obtenu entre une électrode ionisante soumise à une tension positive commandée et une électrode collectrice agencée en regard de ladite électrode ionisante et chargée avec une polarité négative ou reliée à la masse.

## Patentansprüche

1. Verfahren zur Reinigung von Luft in einer Umgebung oder in einem begrenzten Bereich durch eine Behandlungseinheit, welches die folgenden Schritte aufweist:
- einen Schritt, der das Ansaugen eines Luftstroms mit darin suspendierten Partikeln in die Einheit umfasst;
- einen Schritt, der die elektrostatische Filtrierung des Stroms umfasst durch
• das Erzeugen eines elektrischen Feldes mit mindestens einer positiv geladenen Ionisierungselektrode, die in der Lage ist, den Luftstrom mithilfe einer positiven Corona-Entladung unter Generierung von positiven Ionen zu ionisieren, und die in der Lage ist, mithilfe der positiven Corona-Entladung eine gesteuerte Ozonkonzentration in dem Strom zu erzeugen, was während des elektrostatischen Filtrierungsschritts einen Ozonisierungsschritt beinhaltet;
• das elektrische Laden der suspendierten Partikel mit einer im Wesentlichen positiven Ladung mithilfe der positiven Ionen;
• das Auffangen der positiv geladenen, suspendierten Partikel auf mindestens einer Sammelelektrode, die eine Ladung aufweist, die zu der Ionisierungselektrode entgegengesetzt ist, oder die ein Massepotenzial aufweist;
- einen Ionisierungsschritt zum Erzeugen einer Konzentration an negativ geladenen Ionen in dem Luftstrom mithilfe eines Ionisierungsmittels, das in der Lage ist, den Strom nach den Schritten der elektrostatischen Filtrierung und der Ozonisierung aufzubereiten, wobei die Konzentration an negativ geladenen Ionen und die Ozonkonzentration zusammen eine Mischung mit einer deutlich mikrobiziden Wirkung zum Reinigen des Stroms und/oder der Flächen bilden, mit denen er in Kontakt kommen kann;
- einen Schritt, der das Überführen des solchermaßen behandelten Stroms aus der Einheit in eine begrenzte Umgebung beinhaltet.

2. Verfahren nach Anspruch 1, **dadurch gekennzeichnet, dass** die Ozonkonzentration in dem Luftstrom den Schwellengrenzwert (TLV) nicht überschreitet, insbesondere einen 0,1 ppm-Bereich in einer Umgebung, in der sich Personen aufhalten, der auf Basis von acht Arbeitsstunden pro Tag und vierzig Stunden pro Woche berechnet wird.

3. Verfahren nach einem der vorhergehenden Ansprüche, **dadurch gekennzeichnet, dass** es mindestens einen Schritt umfasst, der eine Klimatisierung des Luftstroms beinhaltet, wobei der Schritt der Klimatisierung des Luftstroms nach dem Filtrierungsschritt und nach dem Ionisierungsschritt durchgeführt wird und in der Lage ist, die thermo-hygrometrischen Eigenschaften des Stroms zu regulieren, der in die begrenzte Umgebung mit dem Überführungsschritt eingeleitet werden soll.

4. Verfahren nach einem der vorhergehenden Ansprüche, **dadurch gekennzeichnet, dass** die positiven Ionen, oder die durch diese positiven Ionen positiv geladenen Partikel, welche während des elektrostatischen Filtrierungsschritts nicht abgefangen werden, Oxidverbindungen erzeugen, die mit den Ionen reagieren, die während des negativen Ionisierungsschritts erzeugt wurden, wobei diese Oxidverbindungen in Anwesenheit von Feuchtigkeit wiederum Säureverbindungen erzeugen, die in der Lage sind, die Kontaminierung - insbesondere die bakterielle Verunreinigung - zu reduzieren.

5. Verfahren nach einem der Ansprüche 3 oder 4, **dadurch gekennzeichnet, dass** der Klimatisierungsschritt, welcher nachgeschaltet zu den Filtrierungs- und Ionisierungsschritten durchgeführt wird, es erlaubt, dass der Luftstrom die thermo-hygrometrischen, und insbesondere feuchten Eigenschaften beibehält, welche das Ausbilden der Verbindungen ermöglichen können.

6. Verfahren nach einem der vorhergehenden Ansprüche, **dadurch gekennzeichnet, dass** es mindestens einen Schritt umfasst, der die Verringerung der positiven und/oder negativen Ionen des Luftstroms beinhaltet, wobei dieser Schritt nach den Schritten der elektrostatischen Filtrierung, der Ionisierung, der Ozonisierung und der Klimatisierung und vor dem Schritt, der eine Überführung in die begrenzte Umgebung beinhaltet, auf den Luftstrom angewendet wird.

7. Verfahren nach Anspruch 1, **dadurch gekennzeichnet, dass** mindestens ein Schritt eine Verringerung der Ozonkonzentration beinhaltet, wobei der Ozonverringerungsschritt nach den Schritten der elektrostatischen Filtrierung, der Ionisierung, der Ozonisierung und der Klimatisierung und vor dem Schritt, der eine Überführung in die begrenzte Umgebung beinhaltet, auf den Luftstrom angewendet wird.

8. Verfahren nach Anspruch 1, **dadurch gekennzeichnet, dass** es mindestens einen Schritt umfasst, der die Wiederherstellung des natürlichen Konzentrationsniveaus der Ionen beinhaltet, das in der Luft vorhanden sind, wobei dieser Schritt nach dem Schritt, der eine Verringerung der positiven und/oder negativen Ionen in der Luft und eine Verringerung des Ozons beinhaltet, auf den Luftstrom angewendet wird.

9. Verfahren nach Anspruch 1, **dadurch gekennzeichnet, dass** es mindestens einen Schritt umfasst, der das Mischen des behandelten Luftstroms mit einem Luftstrom beinhaltet, der aus der begrenzten Umgebung und/oder mit einem Luftstrom von außerhalb der begrenzten Umgebung gesaugt wurde.

10. Verfahren nach einem oder mehreren der vorhergehenden Ansprüche, **dadurch gekennzeichnet, dass** der Überführungsschritt ein Verteilen des behandelten Luftstroms über Flächen beinhalten soll, die man reinigen kann.

11. Verfahren nach einem oder mehreren der vorhergehenden Ansprüche, **dadurch gekennzeichnet, dass** der Ozonisierungsschritt zum Teil mithilfe von Ozonisierungsmitteln durchgeführt wird, die dem Filtrierungsmittel nachgeschaltet und in der Nähe des Ionisierungsmittels angeordnet sind, um die mikrobizide Mischung zu erzeugen.

12. Verfahren nach einem oder mehreren der vorhergehenden Ansprüche, **dadurch gekennzeichnet, dass** der Schritt, der das Erzeugen von negativ geladenen Ionen beinhaltet, und der Ozonisierungsschritt für das Erzeugen einer gesteuerten Ozonkonzentration dadurch verwirklicht werden, dass die Gaskomponenten, aus denen der Luftstrom besteht, direkt behandelt werden.

13. Verfahren nach Anspruch 1, **dadurch gekennzeichnet, dass** in der mikrobiziden Mischung die gesteuerte Konzentration an negativen Ionen mindestens 50.000 Ionen pro Kubikzentimeter entspricht.

14. Verfahren nach Anspruch 1, **dadurch gekennzeichnet, dass** der Schritt, der das Erzeugen einer Konzentration an negativ geladenen Ionen beinhaltet, mithilfe der kontinuierlichen, oder diskontinuierlichen, oder konstanten, oder modulierten Ionisierung des Luftstroms durchgeführt wird, und **dadurch gekennzeichnet, dass** der Schritt, der das Erzeugen einer gesteuerten Ozonkonzentration beinhaltet, dementsprechend kontinuierlich, oder diskontinuierlich, oder konstant, oder außerdem moduliert ist, um so eine mikrobizide Mischung mit vorgegebenen Konzentrationsverhältnissen von negativen Ionen und Ozon zu bilden.

15. Verfahren nach Anspruch 1, **dadurch gekennzeichnet, dass** die lokal begrenzte Reinigung nur auf bestimmten Flächen durchgeführt wird, insbesondere auf Gegenständen oder losen Materialien.

16. Verfahren nach Anspruch 6, **dadurch gekennzeichnet, dass** die begrenzte Umgebung aus einer Umgebung besteht, die für den Aufenthalt von Personen geeignet ist, und **dadurch gekennzeichnet, dass** der Verringerungsschritt bewirken soll, dass die Konzentrationen der positiven und/oder negativen Ionen in dem Luftstrom auf Werte gesenkt werden, wie sie im Allgemeinen naturgemäß in der Atmosphäre anzutreffen sind.

17. Verfahren nach Anspruch 7, **dadurch gekennzeichnet, dass** die begrenzte Umgebung aus einer Umgebung besteht, die für den Aufenthalt von Personen geeignet ist, und **dadurch gekennzeichnet, dass** der Verringerungsschritt bewirken soll, dass die Ozonkonzentration auf vorgegebene Werte gesenkt wird, und im Besonderen, dass die Konzentration an O3 auf 200 Mikrogramm pro Kubikmeter gesenkt wird, bezogen auf die durchschnittliche Konzentration pro Stunde und auf 65 Mikrogramm pro Kubikmeter, sowie bezogen auf die durchschnittliche Konzentration über vierundzwanzig Stunden.

18. Vorrichtung für die Reinigung der Luft aus der Umgebung oder einem begrenzten Bereich, insbesondere für die Umsetzung des Verfahrens nach einem oder mehreren-der vorhergehenden Ansprüche, die an eine Behandlungseinheit betriebsbereit angeschlossen werden kann, **dadurch gekennzeichnet, dass** sie umfasst:
- Mittel für die Zwangsumwälzung eines Luftstroms mit suspendierten Teilchen zwischen mindestens einem Ansaugabschnitt und mindestens einem Abgabeabschnitt der Behandlungseinheit;
- Mittel zur elektrostatischen Filtrierung des Stroms mittels der Erzeugung eines gesteuerten elektrischen Felds und einer positiven Corona-Entladung mithilfe von mindestens einer Ionisierungselektrode mit positiver Polarität, die in der Lage ist, positive Ionen zu erzeugen, und die suspendierten Partikel positiv zu laden, um sie auf mindestens einer Sammelelektrode aufzufangen, die in Richtung der Vorwärtsbewegung des Luftstrom der Ionisierungselektrode nachgelagert angeordnet ist, wobei die positive Corona-Entladung in der Lage ist, eine gesteuerte Ozonkonzentration zu erzeugen;
- Ionisierungsmittel, das dem elektrostatischen Filtrierungsmittel in Bezug auf die Richtung der Vorwärtsbewegung des Luftstroms nachgelagert angeordnet ist, und das in der Lage ist, eine Konzentration an negativ geladenen Ionen in dem Strom zu erzeugen, wobei die Konzentration an negativ geladenen Ionen und die Ozonkonzentration zusammen eine Mischung mit einer deutlich mikrobiziden Wirkung zum Reinigen des Stroms und/oder der Flächen bilden, mit denen er in Kontakt kommen kann.

19. Vorrichtung nach Anspruch 18, **dadurch gekennzeichnet, dass** sie ein Überwachungsmittel zum Erfassen der Ozonkonzentration aufweist, die von dem Ozonisierungsmittel emittiert wird.

20. Vorrichtung nach Anspruch 18, **dadurch gekennzeichnet, dass** sie ein Ozon verringerndes Mittel und ein positive und/oder negative Ionen verringerndes Mittel aufweist, die zu dem Ionisierungsmittel nachgelagert angeordnet sind, um das Einführen der jeweiligen Konzentrationen an Ozon und an negativen Ionen in den Luftstrom steuern zu können.

21. Vorrichtung nach Anspruch 18, **dadurch gekennzeichnet, dass** der Strom, der von dem Abgabeabschnitt abgegeben wird, mithilfe eines Überführungsmittels auf Flächen und Gegenstände übertragen wird, die mit der mikrobiziden Mischung gereinigt werden sollen.

22. Vorrichtung nach Anspruch 18, **dadurch gekennzeichnet, dass** das Ionisierungsmittel Elektroden aufweist, die negative Ionen emittieren.

23. Vorrichtung nach Anspruch 18, **dadurch gekennzeichnet, dass** das elektrische Feld zwischen einer Ionisierungselektrode, die einer gesteuerten positiven Spannung ausgesetzt ist, und einer Sammelelektrode erhalten wird, die auf die Ionisierungselektrode ausgerichtet angeordnet ist, und die mit negativer Polarität geladen oder mit einer Masse verbunden ist.
